# EUROPEAN PATENT APPLICATION

(11) **EP 4 005 667 A1**
(43) Date of publication of application: **01.06.2022**
(21) Application number: 20210443.6
(22) Date of filing: 27.11.2020
(51) Int. Cl.: B01J 19/08, B01J 19/12, A61L 2/00, A61L 2/14, A61L 9/22, A61L 9/16, A61L 9/20

(54) **NON-THERMAL PLASMA AIR PURIFIER**

(71) Applicant: Plasma Innova S.A., 11501 San Jose (CR)
(72) Inventor: GONZALEZ, Erick Silesky, 11501 San Jose (CR); CASTILLO, Jose Asenjo, 11501 San Jose (CR); MELENDES, Jaime Mora, 11501 San Jose (CR); SIMON, Juan Carlos Brenes, 11501 San Jose (CR)
(74) Representative: Kohler Schmid Möbus Patentanwälte

(57) **Abstract**

The invention relates to an air purifier (10) for disinfecting or sterilizing ambient air by a cold plasma, comprising a housing (12) (12) with a treatment chamber (20) arranged therein and a plasma unit (26) having a first and a second electrode (32, 34) arranged within the treatment chamber (20) and which are separated from each other by a dielectric (36). The dielectric (36) comprises at least one tubular section (40) which extends in an axial direction with regards to the main flow direction (22) of the air within the treatment chamber (20). The first electrode (32) at least sectionally extends into or extends through each tubular section of the dielectric (36), preferably in an axial direction. The second electrode (34) embraces each tubular section (40) in a ring-shaped fashion such that during operation of the air purifier (10) a dielectric (36) barrier discharge or non-thermal plasma vortex is generated inside each tubular section (40). A fan serves to move the ambient air to be purified through the treatment chamber (20).

## Description

The present invention relates to a non-thermal plasma air purifier for disinfection and/or sterilization of air.

The process of disinfection of biological air contaminants such as viruses, bacteria but also elimination of volatile organic compounds (VOCs) and the like has traditionally been carried out using ultrafiltration and/or chemical processes; among the most used are UV light, filters, chemicals and ionizators.

Filtration is quite expensive and to a certain extent inefficient due to the use of consumables which usually only retain microparticulate matter, and if proper maintenance is not given, conditions are created for incubation and recirculation of viruses, VOC batteries, added to the adverse effects on the environment due to poor handling and maintenance of the filters.

As for chemicals that vaporize and are emitted to the environment, there is a risk of adverse or even toxic effects on humans. Further, most chemicals fail to fully eliminate potentially infectious biological contaminants.

In addition, intentional ozonizers are used to disinfect air, generate and inject ozone into the ambient air to be purified. Ozone is widely used as it reacts about 3000 times faster than chlorine. Its main problem is when the ozone concentration in the air is higher than 240 µg / m3 and is maintained for e.g. more than eight hours, there is a clear risk for human health.

US patent no. 5635059 discloses a plasma air purification system that uses a reactive bed for the generation of the plasma discharge by alternating current in the range of 0.5kHz to 40kHz and flow capacities of 10 CFM (cubic feet per minute) or higher. The problem with this technique lies in the use of an active bed the properties of which degrade over time, making it more expensive and adding a consumable for the user.

Chinese patent CN 101929255 B discloses an air purification and sterilization system for hospital operating rooms, which can rather efficiently kill bacteria and viruses, degrade TVOC, remove peculiar odor and granular spray. The said air purification system has a pulsed plasma reactor. The plasma reactor has a positive electrode and a negative electrode made of different metals.

WO 2009 134 663 A1 discloses a further air purification system, which uses a plasma reactor to treat aerosol particles in a fluid stream that passes through the reactor. The reactor comprises a plasma chamber with a self-cleaning electrode configured to clean debris in the reactor, a carbon pre-filter is also included to filter the air flowing into the reactor chamber and reduce silicone-based contaminants. The problem with this technique lies in the use of filters and consumables.

### OBJECTIVE OF THE INVENTION

The objective of the present invention is to provide a low cost cold plasma air purifier, which allows a reliable disinfection of biological and potentially infectious contaminants at an improved rate and is stackable according to the need of the volume and space to be treated, without affecting human health, human activity and acting directly on the air without the need of filters or other consumables for the users.

### SUMMARY OF THE INVENTION

The **NON-THERMAL PLASMA AIR PURIFIER** according to the invention serves for disinfecting ambient air by a cold plasma vortex. The air purifier can be used, for instance, in closed rooms for protection of health on the job, in schools and so forth and may constitute a further tool against the spread of infections of COVID-19 or other super viruses or bacterias.

The air purifier comprises housing with a treatment chamber arranged therein and a plasma unit having a first and a second electrode arranged within the treatment chamber, which are separated from each other by a dielectric. The dielectric comprises at least one tubular section which extends in an axial direction with regards to the main flow direction of the air within the treatment chamber. The first electrode at least sectionally extends into or extends through each tubular section of the dielectric, preferably in an axial direction. The second electrode, at least sectionally, embraces each tubular section in a ring-shaped fashion such that during use of the air purifier a dielectric barrier discharge (non-thermal plasma vortex) is generated inside each tubular section. The air purifier features a fan for moving the ambient air through the treatment chamber and and back into the ambient atmosphere after its treatment. The air purifier allows to directly expose at least part of the volume flow of the air through the treatment chamber to the dielectric discharge, that is the cold plasma or non-thermal plasma vortex.

This leads to a series of mechanisms that activate the air through the generation of electrons, ions, free radicals, reactive species, among others.

Cold plasma at atmospheric pressure is a relatively new area when it comes to technologies for the disinfection and sterilization of air, water, and surfaces. Although it is true the mechanisms of action as a whole continue to be the object of study due to their complexity and the excellent results obtained, the most recent theories suggest that there are more complex inactivation mechanisms in which different sub-mechanisms of plasma can create a synergistic effect alternating processes within the microorganism, virus or molecule and, therefore, are jointly responsible for the disinfection and sterilization of the air facing the non-thermal plasma vortex generated by the non-thermal plasma reactor.

Among these sub-mechanisms and species that contribute to this complex process of inactivation, disinfection and sterilization are:• UV photons
- Electric field
- Ions
- Reactive oxygen species (ROS)
- Reactive nitrogen species (RNS)
- Free electrons
- Metastable species
- Magnetic fields

Some studies describe that UV or UVC ultraviolet radiation (λ <280 nm) emitted by the dielectric discharge could cause intrinsic photodesorption or damage to the DNA or RNA of bacteria, viruses or other microorganisms present and mixed with the air. Others have reported that the discharges of dielectric barriers are capable of damaging bacteriophage proteins and DNA. There is also talk of the erosion of the microorganism induced by photons that break chemical bonds in the material of the microorganism and lead to the formation of volatile compounds from atoms intrinsic to the microorganism.

Plasma discharges create charged particles and an electric field. It is, therefore, proposed that electrical forces affect the cell membrane, causing electrostatic disruption or at least permeabilization for a very short time. As a consequence, ROS (reactive oxygen species) / RNS (reactive nitrogen species) molecules derived from non-thermal plasma, such as reactive free radicals (NO, OH and superoxide) or strong oxidizing agents (H2O2 and O3), penetrate the microorganism, oxidizing proteins or microbial DNA. ROS / RNS disrupting the integrity of the microbial cell structure by lipid peroxidation, resulting in membrane damage which has also been explored in other studies for non-thermal plasma-derived ROS / RNS.

According to the invention, the first electrode, during operation of the air purifier, is excited by a high ac voltage up to 30 kilovolts peak-pea. This generates a plasma discharge (plasma vortex) against the tubular sections and the second electrode, which is connected to a reference voltage.

The purpose of the electrode made up of copper and titanium is to make the electrode inside the non-thermal plasma reactor resistant to oxidation.

According to a preferred embodiment of the invention, the first electrode, the second electrode and the dielectric each have a frame section which jointly define a flowthrough passage for the main flow of air flowing through the treatment chamber. The frame section can contact the housing of the air purifier in a circumferential direction. By this, the air purifier can be operated at very low noise levels, which allow use in offices, classrooms and so forth. The flowthrough passage allows for a bypass flow of air entering the treatment chamber with respect to the plasma unit. However, this does by no means diminish the efficacy of the air purifier as plasma effects on the air exiting the plasma unit, e. g. the active species like ozone (O₃), electrons, ions and radicals generated in the plasma unit, persist downstream the plasma unit, where the air flows are reunited and mixed.

According to a preferred embodiment of the invention, the opening area A₁ of each tubular section is less than 10 % of the opening area A2 of the flow through passage.

The dielectric may be made in one piece or be designed as a multi-piece part. Preferably, the aforementioned frame section of the dielectric is made from a polymer, e.g. polycarbonate (PC). According to the invention, each tubular section can be made from aluminum dioxide.

According to the invention, there may be at least one UV light source provided upstream the plasma unit in the treatment chamber and/or downstream the plasma unit. The terms upstream and downstream are to be understood with respect to the main direction of flow of the air through the treatment chamber. It needs to be noted that during operation the plasma unit, the cold plasma itself is associated with emission of UV-light. Preferably, there is a first UV-light source provided upstream and a second UV-light source provided downstream. The UV-light provides a further biocidal effect in the air, to allow for a very reliable disinfection and sterilization of the air and it also has a catalytic effect on the reactive species that accumulate inside the chamber. Each UV-light source may comprise UV-LED's (light emitting diodes) for a compact design and improved energy efficiency.

Likewise, the cold plasma or plasma vortex generated by the non-thermal plasma reactor avoids the accumulation of viable microparticles and no substrate or conditions are provided to the viable ones for their growth inside the device (it is self-disinfected).

To prevent an emission of ozone excessive of maximum allowable concentration (MAC), the air purifier, according to a preferred embodiment, has at least one baffle plate for the air flowing out from the treatment chamber which comprises or is made of activated carbon. The function of activated carbon is to catalyze excess ozone to keep air purifier emissions below established and recommended limits.

The aforementioned second UV-light source may be used for promoting the chemical reaction of ozone together with activated carbon:

2O₃ + C → 2 O₂ + CO₂

(O₃ + *h*V_{(240-280 nm)} → O₂ + O)

The CO₂ emission of the air purifier designed for interior use in an office or comparable room as well as the effects thereof on humans is negligible.

According to a further preferred embodiment of the invention, each UV-light source may comprise UV-light emitting diodes (LED's). LED's are available at cost-effective prizes and have a low energy consumption when compared to other known UV-light sources. Also, the LED's allow for a rather simple heat dissipation and are very compact in size.

Each UV-light source may, during use, emit light in the UV-C and/or UV-B spectrum. If there are a first and a second UV-light source provided, the first UV-light sources are preferably different as to the wavelength emitted therefrom. For instance, the one UV-light source may be designed to emit light having a wavelength of around 254 nanometers. The respective other UV-light source may emit UV-light having a frequency of 300-400.

According to a further preferred embodiment of the invention, the dielectric has two tubular sections. The tubular sections are preferably arranged spaced apart from each other and arranged parallel to each other. This simplifies construction of the air purifier.

The first electrode of the plasma unit, according to the invention, is preferably made of copper and titanium. An undesirable oxidation of the first electrode can thereby be prevented. The second electrode according to the invention, is preferably made of copper.

According to the invention, the air purifier and/or the plasma unit comprises a control unit, which may comprise at least one printed circuit board or be formed by one or more printed circuit boards. Each printed circuit board is preferably mounted downstream with respect to at least one of the electrodes. It is needless to say that each of the electrodes is connected in an electrically conductive manner to the control unit.

The fan can, in particular be designed as a radial fan. This ensures a highly efficient and yet low noise level during operation of the air purifier. Further, radial fans are available which have high output and are yet compact in size. This facilitates a modern and delicate design of the air purifier or an air purification system featuring such.

The housing of the air purifier may be made of polycarbonate, polyvinyl chloride or any other suitable housing material.

The plasma unit, in particular all electrically conductive components thereof, are completely arranged within the housing to guarantee the efficiency and at the same time the safety of the air purification process through contact with the non-thermal plasma vortex.

The air purifier, according to the invention, can be parametrized to move up to 40 or even 100 cbm of air per hour. The non-thermal plasma reactor is designed not to have any type of consumable filter and is destined to have a service life to an average of more than 7 years.

Further advantages, features and details of the invention emerge from the description which follows and in which exemplary embodiments of the invention are described in detail with reference to the drawings. The features mentioned in the claims and in the description can then in each case be significant for the invention individually on their own or in any combination.

For a better understanding of the invention, its operating advantages and specific objects attained by its uses, reference is made to the accompanying drawings and descriptive matter in which preferred embodiments of the invention are illustrated.

### BRIEF DESCRIPTION OF THE DRAWINGS

In the drawings
- FIG. 1: is a schematic view showing an embodiment of a air purifier in accordance with the invention;
- FIG. 2: is a schematic view showing the air purifier according to claim 1 in a sectional view; and
- FIG. 3: is an exploded view of the air purifier according to Fig. 2 showing the main parts thereof.

### DESCRIPTION OF THE PREFERRED EMBODIMENT

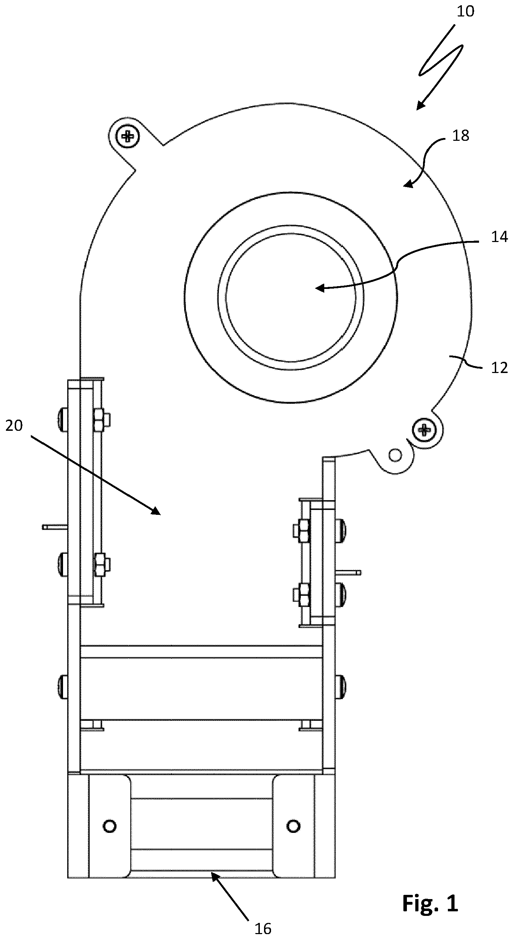

**FIG. 1** shows an air purifier **10** in accordance with the invention. The air purifier 10 serves for disinfecting or sterilizing ambient air by a cold plasma and comprises a housing **12** with an air inlet **14** for the air to be purified and an air outlet **16** for the purified air. The air purifier 10 comprises a blower or fan **18,** which may be designed as a radial fan 18, and a reactor or air treatment chamber **20** arranged within the housing 12.

The housing 12 may be made from polyvinyl chloride, polycarbonate or any other suitable housing 12 material. The treatment chamber 18 is - apart from the air inlet/outlet 14, 16 completely sealed to the outside to ensure the efficiency and at the same time the safety of the air purifier.

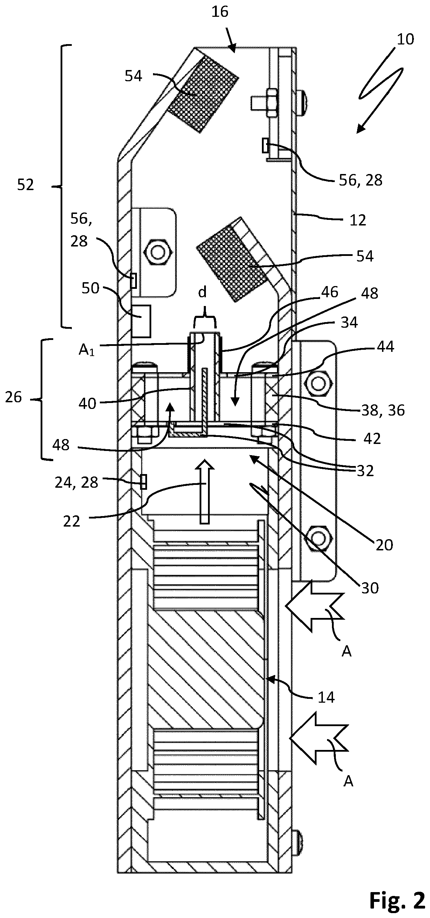

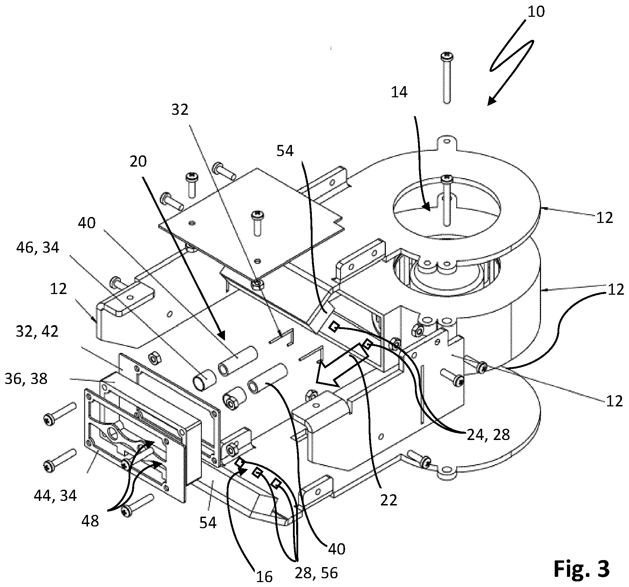

**Fig. 2** shows a sectional view of the air purifier 10 and **Fig.** 3 shows an exploded view of the air purifier 10. The ambient air A to be cleaned flows through the treatment chamber 20 along a main direction of flow **22.**

For disinfection of any organic and potentially infectious matter contained in the air, such as, for instance, viruses, bacteria or prions, there is a first UV-light source **24** and a plasma unit **26** arranged within the treatment chamber 20 of the housing.

The UV light source 24 may comprise several UV-light emitting diodes (LED's) **28** which can, in particular, be arranged on the inner surface **30** of the housing 12. The first UV-light source 24 preferably emits light with a wavelength in the UV-C spectrum, in particular around 254 nanometers. The first UV light source 24 may alternatively or additionally emit light of the UVB and/or the UVA spectrum, e.g. with a wavelength of in between 300 and 400 nanometers.

The plasma unit 26 comprises a first and a second electrode **32, 34** which are both arranged within the treatment chamber 20. The first and second electrode 32, 34 are separated from each other by a dielectric generally denominated **36.** The dielectric 36 may be made in one piece or may be designed in several parts. In the embodiment shown in the Figs., the dielectric 36 comprises at least one frame section **38** and two tubular sections **40,** which extend in an axial direction with regards to the main direction of flow 22 of the air within the treatment chamber 20. The tubular sections 40 of the dielectric 36 are preferably arranged spaced apart from each other and arranged parallel to each other. It is to be understood, that the dielectric may have just one or more than the two tubular sections 40 shown. The tubular sections 40 each have a corresponding opening cross section A₁. The tubular sections 40 preferably have a constant cross-sectional width over the entire length thereof. The internal diameter **d** may be, for instance between 5 to 10 mm, preferably about 6 mm.

The tubular sections 40 of the dielectric 36 can be made e.g. of aluminum dioxide.

The first electrode may likewise have a frame section **42** and at least sectionally extends into or extending through each tubular section of the dielectric 36, preferably in an axial direction. The second electrode 34 likewise may have a frame section **44** and embraces each tubular section 40 with a ring section **46.** The ring section **46** and the frame section 44 of the second electrode 34 may be integrally formed (= in one piece) or may be configured in two parts.

The first electrode 32 is preferably made of copper and titanium, whereas the second electrode 34 is preferably made of copper. The purpose of the electrode 32, 34 made up of copper and titanium is to render the electrode 32, 34 resistants to an undesirable oxidation thereof.

It needs to be noted, that the frame section 38 of the first electrode 32, the frame section of the dielectric 38 and the frame section 44 of the second electrode 34 jointly define a flowthrough passage **48** for the main flow of air flowing through the treatment chamber.

The opening area A1 of each tubular section 40 is less than 20%, particularly preferably less than 10% of the cross-sectional area A2 of the flow through passage.

The air purifier further comprises a control unit **50.** The control unit 50 serves to control all operating parameters of the plasma unit. Also, in particular in the form of one or more printed circuit boards. The control unit can e.g. be mounted downstream at least one of the electrodes 32, 34 and to which both electrodes 32 34 are connected in an electrically conductive manner (not shown in the Figs.). For generation of the dielectric barrier discharge, a high ac voltage is applied to the first electrode 32 while the second electrode 34 is connected to a reference voltage. The first electrode 32 is excited by a high ac voltage up to 30 kilovolts peak-peak and by which a plasma discharge is generated inside the dielectric tubular sections 40 and against the second electrode 34.

Further downstream the electrodes 32, 34 the housing 12 forms an outlet zone **52** comprising the air outlet 16. The outlet zone 52 features at least one, preferably several, baffle plates **54** for the air flowing out from the treatment chamber 20/tubular sections 40. Each baffle plate 54 comprises or is made of activated carbon. The function of activated carbon is to catalyze excess ozone to keep the ozone emissions of the air purifier below established and recommended limits. The chemical equation reads as follows:

2 O₃ + C → 2 O₂ + CO₂

(O₃ + *h*V_{(240-280 nm)} → O₂ + O)

There may be a second UV-light source **56** positioned downstream the electrodes in the outlet zone and by which the reaction of ozone together with activated carbon of the baffle plates 54 may be further promoted. If the UV-light light has a wavelength of approx. 254 nanometers, a further biocidal effect can be realized in the air, reaching disinfection and sterilization levels sufficiently reliably.

During use of the air purifier 10, the air exiting the fan 18 is first exposed to the UV-light emitted by the first (upstream) UV light source 24 to thereby reduce the number of viruses, bacteria and other possible infectants contained in the air.

Only a portion of the volume flow of air per minute through the treatment chamber 20 then actually passes through the tubular sections 40 of the plasma unit 26. It is inside the said tubular sections 40 where a dielectric barrier discharge is generated. The dielectric barrier discharge can be visualized as a vortex of light inside each tubular section 40. The air that passes through the tubular sections is thus exposed to a non-thermal plasma therein which causes a series of mechanisms that activate it through the generation of electrons, ions, free radicals, reactive species, among others. Although the mechanisms of action as a whole continue to be the object of studies the most recent theories suggest that there are more complex inactivation mechanisms in which different sub-mechanisms of cold plasma can create a synergistic effect alternating process within the (biological) cell. These are jointly responsible for the disinfection and sterilization of the air exposed to the dielectric barrier discharge.

The aforesaid sub-mechanisms that contribute to this complex process of inactivation, disinfection and sterilization comprise:
- UV photons
- Electric field
- Ions
- Reactive oxygen species (ROS)
- Reactive nitrogen species (RNS)
- Free electrons
- Metastable species
- Magnetic fields

Some studies describe that UV or UVC ultraviolet radiation (λ <280 nm) could cause intrinsic photodesorption or damage to the DNA or RNA of bacteria, viruses or other microorganisms present and mixed with the air, others have reported that the discharges of dielectric barriers are capable of damaging bacteriophage proteins and DNA (1-2). There is also the hypothesis that the erosion of the microorganism induced by photons that break chemical bonds in the material of the microorganism and lead to the formation of volatile compounds from atoms intrinsic to the microorganism.

Plasma discharges create charged particles and an electric field. It is proposed that electrical forces affect the cell membrane, causing electrostatic disruption or at least permeabilization for a very short time (3). As a consequence, ROS / RNS molecules derived from non-thermal plasma, such as reactive free radicals (NO, OH and superoxide) or strong oxidizing agents (H2O2 and O3), penetrate the microorganism, oxidizing proteins or microbial DNA.. ROS / RNS violating the integrity of the microbial cell structure by lipid peroxidation, resulting in membrane damage which has also been explored in other studies for plasma-derived ROS / RNS not thermal.

Likewise, the non-thermal plasma vortex generated by the non-thermal plasma unit 40 avoids the accumulation of viable microparticles and no substrate or conditions are provided to the viable ones for their growth inside the air purifier. The air purifier 10 is therefore self-disinfecting.

The air purifier 10 can be parametrized to move and disinfect or sterilize up to 100 cbm of air per hour or more. The air purifier 10 described hereinabove and using the non-thermal plasma unit 40 is designed not to use any type of consumable or previous filter, and can have a service life of more than 7 years.

## Claims

1. Air purifier (10) for disinfecting or sterilizing ambient air by a cold plasma, comprising:
• a housing (12) (12) with a treatment chamber (20) arranged therein;
• a plasma unit (26) having a first and a second electrode (32, 34) arranged within the treatment chamber (20), which are separated from each other by a dielectric (36),
∘ the dielectric (36) comprising at least one tubular section (40) which extends in an axial direction with regards to the main flow direction (22) of the air within the treatment chamber (20);
∘ the first electrode (32) at least sectionally extending into or extending through each tubular section (40) of the dielectric (36), preferably in an axial direction, and the second electrode (34) embracing each tubular section (40) in a ring-shaped fashion such that during operation of the air purifier (10) a dielectric (36) barrier discharge is generated inside each tubular section (40); and
• a fan (18) for moving the ambient air to through the treatment chamber (20).

2. The air purifier (10) according to claim 1, **characterized in that** the first electrode (32), the second electrode (34) and the dielectric (36) each have a frame section (38, 42, 44) which jointly define a flowthrough passage (48) for the main flow of air flowing through the treatment chamber (20).

3. The air purifier (10) according to claims 1 or 2, **characterized in that** the plasma unit (26) comprises a control unit (50), in particular in the form of a printed circuit board, which is mounted downstream at least one of the electrodes (32, 34) and to which both electrodes (32, 34) are connected in an electrically conductive manner.

4. The air purifier (10) according to any one of the preceding claims, **characterized in that** there is at least one UV light source (24, 56) provided within the treatment chamber (20) which is spaced from each tubular section (40) of the dielectric (36).

5. The air purifier (10) according to any one of the preceding claims, characterized that there is at least one baffle plate (54) for the air flowing out from the treatment chamber (20) which comprises or is made of activated carbon.

6. The air purifier (10) according to claim 1, wherein the dielectric (36) has two tubular sections (40), which are preferably arranged spaced apart from each other and arranged parallel to each other.

7. The air purifier (10) according to any one of the preceding claims, wherein the first electrode (32) is made of copper and titanium, whereas the second electrode (34) is made of copper.

8. The air purifier (10) according to any one of the preceding claims, wherein the UV light source (24) is mounted upstream the first electrode (32).

9. The air purifier (10) according to any of the preceding claims, wherein the fan (18) is designed as a radial fan (18).

10. The air purifier (10) according to any one of the preceding claims, wherein the opening area A₁ of each tubular section (40) is less than 10 % of the opening area A₂ of the flow through passage (48).

11. The air purifier (10) according to any one of the preceding claims, **characterized in that** the dielectric (36) and/or the tubular section (40) thereof is made from aluminum dioxide.

12. The air purifier (10) according to any of the preceding claims, **characterized in that** the UV light source (24, 56) emits UV-light having a wavelength of 256 nm and/or in between 300 nm and 400 nm.

13. The air purifier (10) according to any one of the preceding claims, **characterized in that** air purifier (10) is parametrized to move up to 100 cbm of air per hour.

14. The air purifier (10) according to any one of the preceding claims, **characterized in that** the housing (12) is made of polycarbonate or polyvinylchloride or a fiber-reinforced resin.
